(19) Europäisches Patentamt European Patent Office Office européen des brevets

(11) **EP 3 743 435 B1**

(12) **EUROPEAN PATENT SPECIFICATION**

(45) Date of publication and mention of the grant of the patent:
**09.08.2023 Bulletin 2023/32**

(21) Application number: **19743255.2**

(22) Date of filing: **23.01.2019**

(51) International Patent Classification (IPC):
**C07K 14/00** (2006.01)   **C07K 19/00** (2006.01)
**C12N 11/02** (2006.01)   **C12Q 1/68** (2018.01)
**G01N 21/64** (2006.01)   **G01N 33/536** (2006.01)
**C12Q 1/6804** (2018.01)   **C12Q 1/6841** (2018.01)

(52) Cooperative Patent Classification (CPC):
(C-Sets available)
**C12N 9/22; C12Q 1/6804; C12Q 1/6841;**
C07K 2319/09; C07K 2319/60; C07K 2319/735;
C12N 2740/16043                    (Cont.)

(86) International application number:
**PCT/US2019/014666**

(87) International publication number:
**WO 2019/147611 (01.08.2019 Gazette 2019/31)**

(54) **SYSTEM AND METHOD FOR INDUCING CLUSTERS OF GENE REGULATORY PROTEINS TARGETED TO SPECIFIC GENOMIC LOCI**

SYSTEM UND VERFAHREN ZUR INDUKTION VON CLUSTERN VON GENREGULATORISCHEN PROTEINEN, DIE AUF SPEZIFISCHE GENOMISCHE LOCI GERICHTET SIND

SYSTÈME ET PROCÉDÉ D'INDUCTION DE GROUPES DE PROTÉINES RÉGULATRICES GÉNIQUES CIBLÉES SUR DES LOCI GÉNOMIQUES SPÉCIFIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.01.2018   US 201862621182 P**
**20.09.2018   US 201862734063 P**

(43) Date of publication of application:
**02.12.2020   Bulletin 2020/49**

(73) Proprietor: **The Trustees Of Princeton University Princeton NJ 08544 (US)**

(72) Inventors:
• **BRANGWYNNE, Cliff**
**Hopewell, NJ 08525 (US)**
• **SHIN, Yongdae**
**Princeton, NJ 08540 (US)**
• **BRACHA, Dan**
**Princeton, NJ 08540 (US)**

(74) Representative: **Brand Murray Fuller LLP**
**50 Eastcastle Street**
**London W1W 8EA (GB)**

(56) References cited:
**WO-A1-2017/205837       WO-A2-2016/011070**
**US-A1- 2017 219 596       US-A1- 2017 355 977**
**US-A1- 2018 251 497**

• **LAUREN R POLSTEIN ET AL: "A light-inducible CRISPR-Cas9 system for control of endogenous gene activation", NATURE CHEMICAL BIOLOGY, vol. 11, no. 3, 9 February 2015 (2015-02-09), pages 198-200, XP055194413, ISSN: 1552-4450, DOI: 10.1038/nchembio.1753**
• **SHIN YONGDAE ET AL: "Spatiotemporal Control of Intracellular Phase Transitions Using Light-Activated optoDroplets", CELL, ELSEVIER, AMSTERDAM NL, vol. 168, no. 1-2, 29 December 2016 (2016-12-29), pages 159-171, XP029882177, ISSN: 0092-8674, DOI: 10.1016/J.CELL.2016.11.054**

- CHEN et al.: "Dynamic Imaging of Genomic Loci in Living Human Cells by an Optimized CRISPR/Cas System", Cell, vol. 155, 19 December 2013 (2013-12-19), pages 1479-1491, XP028806611, DOI: 10.1016/j.cell.2013.12.001
- SHIN et al.: "Liquid Nuclear Condensates Mechanically Sense and Restructure the Genome", Cell, vol. 175, no. 6, 29 November 2018 (2018-11-29), pages 1481-1491, XP085544101, DOI: 10.1016/j.cell.2018.10.057
- XUE et al.: "Live- Cell Imaging of Chromatin Condensation Dynamics by CRISPR", iScience, vol. 4, 29 June 2018 (2018-06-29), pages 216-235, XP055700100,
- NIHONGAKI et al.: "CRISPR-Cas9-based Photoactivatable Transcription System", Chemistry & Biology, vol. 22, 19 February 2015 (2015-02-19), pages 169-174, XP055282700, DOI: 10.1016/j.chembiol.2014.12.011
- SHIN et al.: "Spatiotemporal Control of Intracellular Phase Transitions Using Light-Activated OptoDroplets", Cell, vol. 168, 12 January 2017 (2017-01-12), pages 159-171, XP029882177, DOI: 10.1016/j.cell.2016.11.054
- TANENBAUM et al.: "A Protein-Tagging System for Signal Amplification in Gene Expression and Fluorescence Imaging", Cell, vol. 159, 23 October 2014 (2014-10-23), pages 635-646, XP029084861, DOI: 10.1016/j.cell.2014.09.039
- CHEN TINGJUN ET AL: "Chemically Controlled Epigenome Editing through an Inducible dCas9 System", JOURNAL OF THE AMERICAN CHEMICAL SOCIETY, vol. 139, no. 33, 23 August 2017 (2017-08-23), pages 11337-11340, XP055921375, ISSN: 0002-7863, DOI: 10.1021/jacs.7b06555

(52) Cooperative Patent Classification (CPC): (Cont.)

C-Sets
C12Q 1/6804, C12Q 2523/319, C12Q 2537/162,
C12Q 2563/119, C12Q 2563/131, C12Q 2565/601;
C12Q 1/6841, C12Q 2523/319, C12Q 2537/162,
C12Q 2563/119, C12Q 2563/131, C12Q 2565/601

**Description**

CROSS-REFERENCE TO RELATED APPLICATIONS

This application claims the benefit of U.S. Provisional Application Nos. 62/621,182, filed January 24, 2018, and 62/734,063, filed September 20, 2018.

STATEMENT REGARDING FEDERALLY SPONSORED RESEARCH OR DEVELOPMENT

This invention was made with government support under Grant No. DA040601 awarded by the National Institutes of Health. The United States government has certain rights in the invention.

TECHNICAL FIELD

This relates generally to the induced clustering of proteins, and more particularly, to inducing clusters of gene regulatory proteins targeted to specific genomic loci.

BACKGROUND OF THE INVENTION

Liquid-liquid phase separation (LLPS) is a fundamental mechanism for organizing the contents of living cells. LLPS is now recognized as important for driving assembly of a wide range of membrane-less condensates, including cytoplasmic structures such as germ (P) granules, stress granules, miRISC assemblies, and synaptic scaffolds. LLPS also appears to underlie nuclear body biogenesis, including nucleoli, and likely many others. Associated liquid-to-solid phase transitions are also implicated in various diseases of pathological protein aggregation. Intracellular phase transitions arise from weak, multivalent interactions, often mediated by intrinsically disordered proteins/regions (IDPs/IDRs), which are closely related to low complexity sequences and prion-like domains.

Phase transitions within the nucleus are particularly interesting, since nuclear condensates must directly interact with chromatin, and thus potentially control its organization and gene expression. Consistent with this, the assembly and dynamics of nuclear condensates such as Cajal bodies, nucleoli, and speckles appear to impact chromatin architecture. Moreover, recent studies suggest that phase separation of heterochromatin protein 1 (HP1) underlies formation of heterochromatin dense, transcriptionally silent regions of the genome. Phase separation is also linked to active regions of the (euchromatic) genome. For example, nucleoli assemble at transcriptionally active ribosomal DNA loci, in a transcription-dependent manner. Phase separation has also been recently implicated in driving gene activation through nanoscale transcriptional condensates assembled at enhancer rich gene clusters.

Despite the growing number of studies suggesting the importance of phase separation for nuclear organization, the biophysics of these processes remains mysterious. From a biophysical perspective, the genome is a complex viscoelastic polymer matrix, whose 3D architecture and mechanics are key factors governing gene expression. On the one hand, gene activation through transcriptional condensates has been proposed to arise from droplets pulling together distal regions of the genome, through a still uncharacterized mechanism. This could be important for promoting enhancer-promoter interactions, that in some cases may be many kilobases away from one another, or even reside on different chromosomes. Surprisingly, this is conceptually similar to the proposed function of HP1, which may drive heterochromatin formation by condensing the genome into compact, transcriptionally silent foci. In any case, it is unclear how phase separation could give rise to mechanical forces capable of restructuring the genome, while providing the specificity required to coordinate associated gene activity. Moreover, the way in which the mechanics of the genome may in turn impact phase separation, as a potential feedback mechanism for controlling gene expression, is still completely unknown.

These fundamental questions underscore the need for a quantitative understanding of the forces at play in mediating interactions between condensates and specific regions of the genome, and their consequences for the dynamics of phase separation and chromatin organization. One of the key challenges to addressing this problem is the lack of tools for controlling phase transitions in living cells.

Several genome targeting techniques, including CRISPR-Cas9 based ones, have been applied to genome and epigenome engineering. However, none of these techniques allows for controlled alteration of nuclear architecture at the scale of up to a micron or more. Meanwhile, recent optogenetic developments can induce large scale assembly of proteins in the nucleus, but their localization is random and not controlled with regard to underlying genomic environment, limiting their application for functional studies of biological processes in the nucleus.

Thus, a system and method that combine advantages of genome targeting and optogenetics to enable the controlled delivery of protein clusters at specific genomic loci is desirable.

WO 2016/011070 discloses methods, compositions, and kits for imaging a polypeptide of interest and site-specific transcriptional regulation of one or more genetic elements.

Yongdae Shin et al (Cell. 2017 Jan 12;168(1-2):159-171.e14. doi: 10.1016/j.cell.2016.11.054. Epub 2016 Dec 29. PMID: 28041848; PMCID: PMC5562165) focusses on spatiotemporal control of intracellular phase transitions using light-activated optoDroplets and discloses an optogenetic platform that uses light to activate IDR-mediated phase transitions in living cells.

BRIEF SUMMARY OF THE INVENTION

The present disclosure is drawn to a system and method of inducing clusters of gene regulatory proteins targeted to specific genomic loci. The system includes a first structure having a Cas-based genomic targeting protein fused or attached to one or more sequences, each of which may be a light-sensitive receptor, a chemical-sensitive receptor, a light sensitive oligomerization protein or a non-light sensitive dimerization module. The system may also include a second structure, which includes a cognate partner of the light- or chemical-sensitive receptor protein or a dimerization domain complementary to the dimerization module, where the cognate partner or complementary dimerization domain is fused to at least one transcriptional regulatory protein having a full length or truncated low complexity or intrinsically-disordered protein region. The first structure may include dCas9 fused to SunTag, which is attached to another construct having a single chain variable fragment antibody fused to a superfolding variant of GFP (sfGFP) and iLID, where the single chain variable fragment antibody is a cognate for SunTag. Advantageously, the first structure may include at least one reporter protein. Also advantageously, the cognate partner or complementary dimerization domain may be fused to full length or truncated BRD4, FUS, or TAF15. The first structure may include repeating sequences.

In a first aspect, the invention relates to a system for the controlled clustering of gene regulatory proteins at specific target genomic loci, comprising:

a first structure comprising;

(i) a first segment having a Cas-based genomic targeting protein fused or attached to one or more first sequences, each first sequence including at least one sequence selected from the group consisting of a light-sensitive receptor, a chemical-sensitive receptor, and a light-sensitive oligomerization protein; or

(ii) a first segment having a Cas-based genomic targeting protein fused or attached to one or more genes encoding at least one protein sensitive to at least one wavelength of light and a second segment fused to the first segment, the second segment having at least one sequence selected from the group consisting of a low complexity sequence (LCS), an intrinsically disordered protein region (IDR), a synthetic or natural nucleic acid binding domain, and at least one repeatable sequence, wherein the repeatable sequence comprises a linker fused to at least one additional gene encoding at least one protein sensitive to at least one wavelength of light; and

a second structure comprising a second sequence selected from the group consisting of a cognate partner of the light-sensitive receptor protein, a cognate partner of the chemical-sensitive protein, and a dimerization domain complementary to a dimerization module, the second sequence being fused to at least one transcriptional regulatory protein having a full length or truncated low complexity or intrinsically disordered protein region,

wherein the protein system is configured for targeted condensation at the specific target genomic loci by exposing the protein system to at least one predetermined wavelength of light, a chemical to which the chemical-sensitive receptor is sensitive, or a combination thereof.

In a second aspect, the invention relates to an in vitro method for the controlled clustering of gene regulatory proteins at the specific target genomic loci, comprising the steps of:

(a) providing at least one specific genomic target loci and complementary single-guide RNA;
(b) providing a protein system in a living cell, the system including either:

(i) a first segment having a Cas-based genomic targeting protein fused or attached to one or more sequences, each sequence including a light-sensitive receptor, chemical-sensitive receptor, oligomerization protein, or a combination thereof; or

(ii) a first segment having a Cas-based genomic targeting protein fused or attached to at least one gene encoding at least one protein sensitive to at least one wavelength of light; and a second segment fused to the first segment, the second segment having at least one sequence selected from the group consisting of a low complexity sequence (LCS); an intrinsically disordered protein region (IDR); a synthetic or natural nucleic acid binding domain; and at least one repeatable sequence, the repeatable sequence comprising a linker fused to at least one additional gene encoding at least one protein sensitive to at least one wavelength of light; and

(c) inducing targeted condensation at the specific target genomic loci by exposing the protein system to at least one predetermined wavelength of light, a chemical to which the chemical-sensitive receptor is sensitive, or a combination thereof.

Preferred features of the invention are set out in the dependent claims herein.

The disclosed method involves providing at least one specific genomic target loci and complementary single-guide RNA, along with a protein system in a living cell. The protein system should have one of two configurations: (i) a first segment having a Cas-based genomic targeting protein fused or attached to one or more sequences, each sequence including a light-sensitive receptor, chemical-sensitive receptor, oligomerization protein, or a combination thereof; or (ii) a first segment having a Cas-based genomic targeting protein fused or attached to at least one gene encoding at least one protein sensitive to at least one wavelength of light; and a second segment fused to the first segment, the second segment having at least one sequence selected from the group consisting of a low complexity sequence (LCS); intrinsically disordered protein region (IDR); a synthetic or natural nucleic acid binding domain; and at least one repeatable sequence, the repeatable sequence comprising a linker fused to at least one additional gene encoding at least one protein sensitive to at least one wavelength of light.

The method then involves inducing targeted condensation at the specific target genomic loci by exposing the protein system to at least one predetermined wavelength of light, a chemical to which the chemical-sensitive receptor is sensitive, or a combination thereof, which may include a wavelength between 450 nm and 495 nm.

The method may optionally utilize a protein system also includes a second segment having a cognate partner of the light-sensitive receptor protein fused to at least one gene regulatory protein having a full length or truncated low complexity or intrinsically-disordered protein region, or other folded proteins known to promote self-interactions.

The method may also include sensing or measuring the targeted condensation to detect the mechanical state of the genome, which may include includes detecting the presence of tightly condensed heterochromatin.

The induction of targeted condensation may be configured to induce mechanical stresses in the genome, to relax mechanical stresses in the genome, and/or to alter mechanical stresses in the genome. Further, the induction of targeted condensation may include inducing enhancer-promoter interactions, inducing super enhancer clusters, or actuating related types of proximity-dependent gene regulatory elements.

The method may include the use of a system having a first protein system associated with a first genomic locus, and a second protein system associated with a second genomic locus, and wherein exposing the first and second protein systems to the at least one wavelength of light causes droplets to form, then coalesce together. In some of those approaches, when the droplets are formed, the center-to-center separation of the two droplets is less than 3 microns.

The method may include the use of a first structure having a first construct comprising dCas9 fused to SunTag attached to a second construct comprising a single chain variable fragment antibody fused to a superfolding variant of GFP (sfGFP) and iLID where the single chain variable fragment antibody is a cognate for SunTag.

The method may also include allowing the first structure to serve as a multimeric protein scaffold for binding of the second structure; and triggering liquid-liquid phase separation by exposing the first structure to at least one predetermined wavelength of light and causing the first and second structure self-assemble into a multimeric protein complex.

BRIEF DESCRIPTION OF DRAWINGS

The invention will be described hereinafter with reference to non-limitative examples, which are provided for explanatory, non-limitative purposes in the accompanying drawings. These drawings illustrate different aspects and embodiments of this invention and, where appropriate, the structures, components, materials and/or similar elements are indicated in the different figures with similar reference numbers.

Figure 1 is a diagrammatic illustration of a disclosed system prior to the induction of clustering.
Figs. 2A and 2B are a fluorescence images illustrating the impact of the use of single guide RNA (2A) and non-use of single guide RNA (2B) in the pre-seeding process.
Figure 3 is a flowchart describing a disclosed method.
Figures 4A-4C are diagrammatic illustrations of system configurations.
Figure 5 is a diagrammatic illustration of the binding of the first and second structures of a disclosed system.
Figure 6 is a diagrammatic illustration of clustering of a disclosed system.

DETAILED DESCRIPTION OF THE INVENTION

Disclosed is a composition of matter, specifically a set of engineered protein constructs. The system has modular aspects, including at least two layers - genome targeting and phase separating modules. Certain systems have modular aspects with three layers: genome targeting, light sensitive and phase separating modules. One example system builds on the well-characterized genome targeting technique (e.g., a nuclease-deficient Cas9 (dCas9) fused to repeats of epitopes such as SunTag), the light sensitive module involves a single-chain variable fragment (scFv) antibody fused to a light-responsive receptor protein (e.g., an engineered LOV2-ssrA that was named iLID) and the phase separation module consisting of the receptor's cognate partner (e.g., sspB protein) fused to transcriptional regulatory proteins with intrinsically disordered regions. Protein constructs can be delivered into a variety of biological systems including mammalian cell cultures by standard transfection methods or viral delivery.

As used herein, "fused" means covalently bound.

As used herein, "attached" means non-covalently bound.

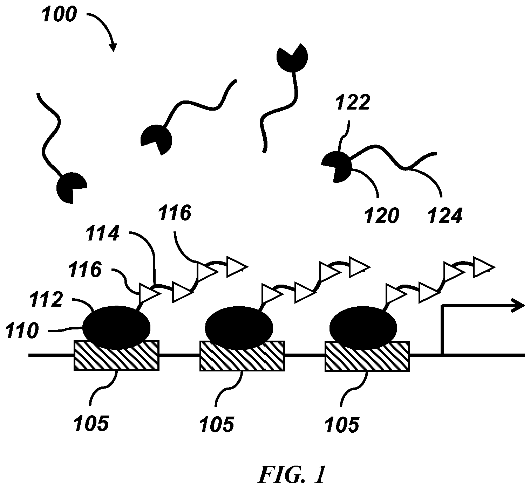

Referring to Fig. 1, a system 100 for the controlled clustering of gene regulatory proteins at specific target genomic loci 105 is shown. The system 100 includes a first structure 110, which may utilize a first construct comprising a Cas-based genomic targeting protein 112 fused or attached to one or more first sequences 116. In some embodiments, the one or more first sequences may include a repeating first sequence. In Fig. 1A, the Cas-based genomic targeting protein 112 may be fused or attached to, e.g., an enabling scaffolding 114, such as SunTag.

In certain embodiments, the Cas-based genomic targeting protein 112 may be enzymatically dead Cas9 (dCas9), such as *S. thermophilus* or *S. pyogenes* dCas9, although other known Cas-based proteins can be utilized. As is known in the art, the type II CRISPR system uses an endonuclease (Cas9) guided by a single guide RNA (sgRNA) that specifically hybridizes and induces a double-stranded break (DSB) at complementary genomic sequences. The use of dCas9 allows the system to targeting genomic DNA without cleaving.

The one or more first sequences 116 may include a light-sensitive receptor, a light-sensitive oligomerization protein, and a chemically-sensitive receptor. Such receptors, proteins, and dimerization modules are known in the art, and may include but are not limited to phytochromes such as the five phytochromes in Arabidopsis thaliana, phyA to phyE, cryptochromes such as CRY2, or Light-Oxygen-Voltage Sensing (LOV) domain-based approaches such as iLID, chemically induced dimerization (CID) modules such as FKBP, or non-stimulus responsive dimerization pairs such as SH3/PRM or SUMO/SIM systems.

The one or more first sequences 116 may also include other elements, including but not limited to, a single chain variable fragment (scFv) antibody, and one or more reporter tags. While any known reporter tags (such as, but not limited to, GFP, mCherry, etc.) are envisioned, preferred embodiments utilize a superfolding variant of GFP (sfGFP). The first structure may include a first construct of dCas9 fused to SunTag, where the SunTag is attached to multiple first sequences, where each first sequence is a second construct comprising a scFv antibody, a reporter tag, and a light-responsive receptor protein such as iLID. In some embodiments, the scFv is a cognate for SunTag.

A single first construct may be fused or attached to a single second construct. A single first construct may be fused or attached to a two second constructs. A single first construct may be fused or attached to a three second constructs. A single first construct may be fused or attached to four or more second constructs. A single first construct may be fused or attached to between 1 and 20 second constructs, preferably between 2 and 15 second constructs, and more preferably between 3 and 12 second constructs, and more preferably between 3 and 6 second constructs.

Still referring to Fig. 1, the system 100 may also include a second structure 120. Second structure 120 may include a second sequence 122, where the second sequence 122 can be a cognate partner of the light- or chemical-sensitive receptor protein or a dimerization domain complementary to the dimerization module. These may include, but are not limited to sspB, phytochrome interacting factor (PIF) such as PIF6, a cryptochrome-interacting basic-helix-loop-helix (CIB) such as CIB1.

The second sequence 122 may be fused to at least one transcriptional regulatory protein 124 having a full length or truncated low complexity or intrinsically-disordered protein region, or to other folded proteins known to promote self-interactions, such as the PTB/PDZ system. In some embodiments, the second sequence may be fused to, for example, full length or truncated BRD4, FUS, or TAF15.

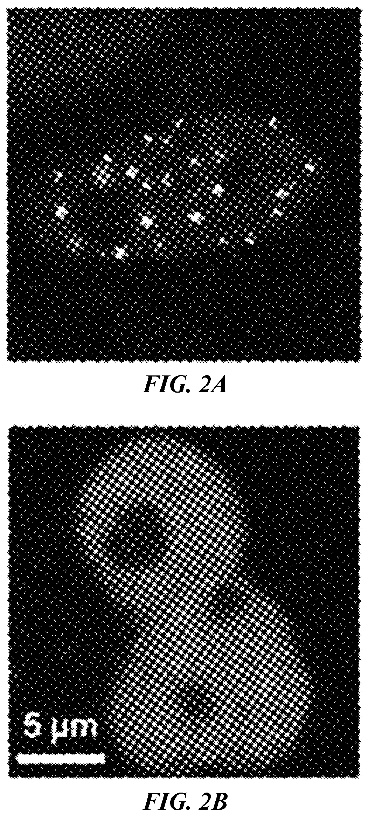

The first structure is generally pre-seeded at one or more target genomic sites 105 through complementary single-guide RNA (sgRNA, not shown), as is known in the art. Further, where the first structure includes a reporter tag, the reporter tag may be used to confirm pre-seeding. For example, pre-seeding at telomeres in a HEK293 cell was accomplished by expressing dCas9-SunTag, scFv-sfGFP-iLID, and sgRNA for telomeres. Fig. 2A provides an image of fluorescence signals from the reporter tags when the system utilized telomere-targeting sgRNA. As can be seen, there is significant localized fluorescence at discrete locations in the cell, indicating pre-seeding has occurred. Fig. 2B provides an image of the fluorescence signals from the reporter tags in systems that do not utilize telomere-targeting sgRNA. As seen, the fluorescence is not localized at specific locations, but rather is broadly spread throughout the cell, indicating pre-seeding has not occurred.

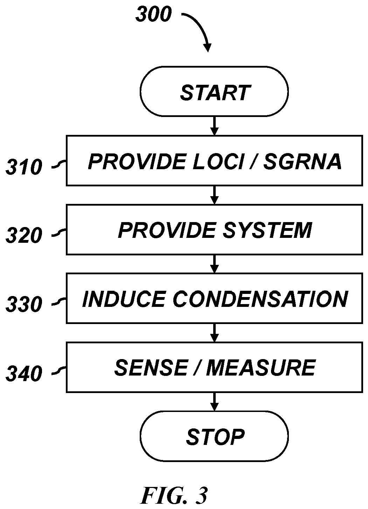

The disclosed method for the controlled clustering of gene regulatory proteins at the specific target genomic loci can be described with reference to Figs. 3-6. Referring to Fig. 3, the method 300 begins by providing 310 at least one specific genomic target loci and complementary single-guide RNA. The method also requires providing 320 a protein system in a living cell. One of skill in the art will recognize that these first steps may occur in any order.

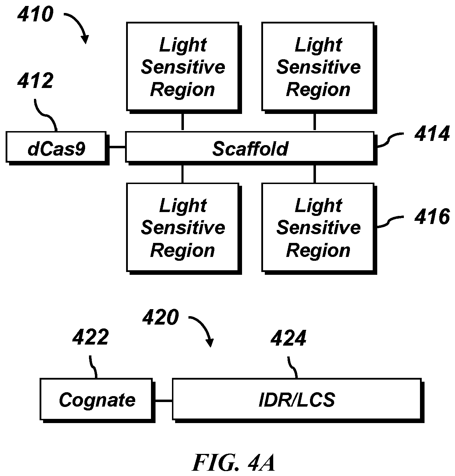

The provided protein system may follow one of two basic configurations, which can be seen in reference to Figs. 4A-4C.

Referring to Fig. 4A, configuration 1 is a system having a first segment 410 that includes a Cas-based genomic targeting protein 412 (shown generically as "dCas9") fused or attached to one or more sequences 416, where each sequence includes a light-sensitive receptor, chemical-sensitive receptor, or oligomerization protein (shown generically as "light sensitive region").

Configuration 1 may also include a second segment 420 that includes a cognate partner 422 of the light- or chemical-sensitive receptor protein (shown generically as "cognate") fused to at least one gene regulatory protein 424 having a full length or truncated low complexity or intrinsically-disordered protein region, or other folded proteins known to promote self-interactions (shown generically as "IDR/LCS").

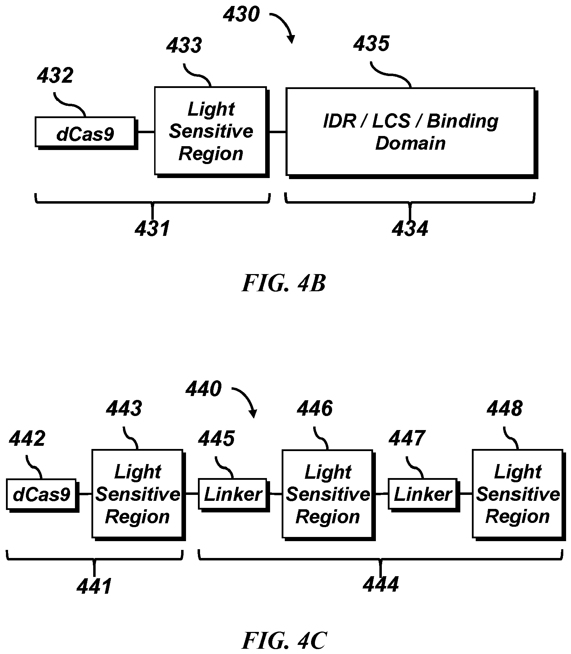

Referring to Figs. 4B and 4C, configuration 2 is a system 430, 440 having a first segment 431, 441 that includes a Cas-based genomic targeting protein 432, 442 (shown generically as "dCas9") fused or attached to at least one gene encoding one or more proteins 433, 443 sensitive to at least one wavelength of light or sensitive to at least one chemical (shown generically as "light sensitive region"). The system also includes a second segment 434, 444 fused or attached to the first segment. The second segment includes one or more of the following sequences: a low complexity sequence (LCS) (see, e.g., 435), an intrinsically disordered protein region (IDR) (see, e.g., 435), a synthetic or natural nucleic acid binding domain (see, e.g., 435) (shown generically as "IDR / LCS / Binding Domain"), and/or one or more repeatable sequences, where the repeatable sequence includes a linker 445, 447 (shown generically as "linker") fused to at least one additional gene encoding at least one protein 446, 448 sensitive to at least one wavelength of light or sensitive to at least one chemical (shown generically as "light sensitive region"). The number of repeats may be between 1 and 20. Preferably, the number of repeats is between 2 and 9, and more preferably the number of repeats is between 3 and 5.

Once the target loci, sgRNA, and protein system have been provided, targeted condensation may be induced 330 at the specific target genomic loci by exposing the protein system to at least one predetermined wavelength of light and/or chemical to which the chemical-sensitive receptor is sensitive. As is understood by one of skill in the art, the wavelength is one appropriate for the light sensitive receptors or proteins used in the system. The predetermined wavelength of light may include a wavelength between 450 nm and 495 nm.

For systems utilizing configuration 1, upon exposure to light, self-interactions among proteins targeted to different genomic elements 105 causes droplets to form, and potentially induce desirable rearrangements to genomic architecture.

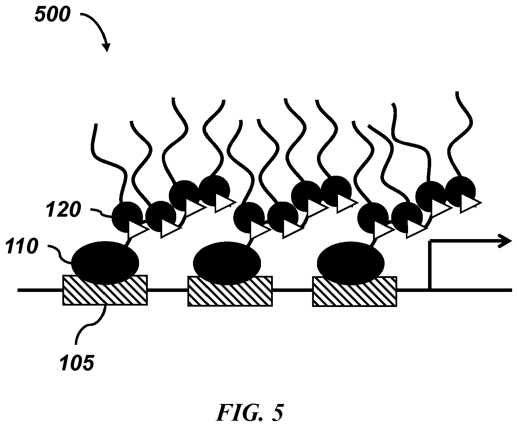

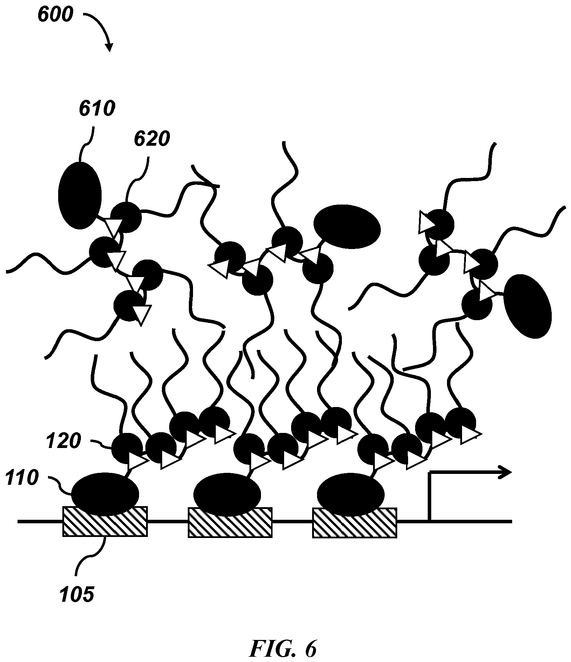

As seen in Fig. 5, upon exposure to light, a cognate partner of a light-sensitive receptor protein that may be present in a second structure 120, will attach to the light-sensitive receptor in a first structure 110. Similarly, upon exposure to a particular compound, a cognate partner of a chemical-sensitive receptor in a second structure will attach to the chemical-sensitive receptor in a first structure. This process causes a droplet to form. As seen in Fig. 6, as exposure to the light continues, additional first structures 610 attached to second structures 620, are drawn into the vicinity of the droplet formed from first structure 110 and second structure 120, causing the size of the droplet to increase. In non-light/chemical sensitive examples, a similar phase separation/clustering would occur, but without the need for inducing attachment of self-interacting modules with light or chemicals. For configuration 1, light-activation could be used to turn on intermolecular interactions, rather than recruit interacting modules, for example using Cry2.

Where the system includes a first structure that can serve as a multimeric protein scaffold (See Fig. 4A, which utilizes scaffold 414) for binding a second structure, and is configured to allow such to occur, liquid-liquid phase separation may be triggered by exposing the first structure to at least one predetermined wavelength of light and causing the first and second structure to self-assemble into a multimeric protein complex.

In certain embodiments, inducing targeted condensation at a specific target genomic locus may induce mechanical stresses in the genome, relax mechanical stresses in the genome, and/or otherwise alter mechanical stresses in the genome.

In certain embodiments, inducing targeted condensation at a specific target genomic locus may involve inducing enhancer-promoter interactions, inducing super enhancer clusters, and/or actuating related types of proximity-dependent gene regulatory elements, or silencing genes through compaction and heterochromatization.

Additional protein systems associated with additional genomic loci may also be utilized. For example, in embodiments where the system includes a first protein system associated with a first genomic locus, and a second protein system associated with a second genomic locus, exposing the protein systems to the at least one wavelength of light may cause droplets to form, which may then coalesce together. In preferred embodiments, when the droplets are formed, the center-to-center separation of two droplets is less than 10 microns, more preferably less than 5 microns, and still more preferably less than 3 microns.

Optionally, the method may also include sensing or measuring 340 the targeted condensation to detect the mechanical state of the genome. This may include detecting the presence of tightly condensed heterochromatin.

Using this approach, it can be shown that various IDPs from various nuclear proteins, including but not limited to BRD4, FUS, and TAF15, phase separate into liquid condensates that mechanically exclude chromatin as they grow, and preferentially form in low-density euchromatic regions. The condensates cannot be driven to form within dense satellite heterochromatin, nor do they mix with heterochromatic telomeres, but when specifically targeted to these regions, can cause them to be mechanically pulled together through surface tension-driven coalescence. A minimal physical model explains this preference for droplet condensation within euchromatin due to the lower mechanical energy cost of droplets deforming softer euchromatic regions. These findings suggest that nuclear condensates can function as mechano-active chromatin filters, causing distal targeted genomic elements to be pulled together, while mechanically excluding non-specific background components of the genome.

To study phase behavior within the nucleus, the disclosed platform, termed CasDrop, can induce localized condensation of liquid droplets at specific genomic loci. Inspired by the way native subnuclear assemblies form, the local concentration of transcriptional regulators and other nuclear proteins are controlled, by programmable seeding to achieve this, the CasDrop system is modularized with three components that allow for (1) pre-seeding, (2) optogenetic molecular assembly and (3) oligomerization-based phase separation. Taking advantage of well-characterized CRISPR-Cas9 technology for programmable targeting to specific genomic loci, one example was created from enzymatically dead Cas9 (dCas9) fused to SunTag (ST). The second construct linking genomic pre-seeding to optogenetic assembly involves single-chain variable fragment (scFv) antibody, cognate for the ST, fused to superfolding GFP (sfGFP) and the optogenetic dimerization protein iLID. When co-expressed in cells, these two constructs can self-assemble into a multimeric protein complex (hereafter referred to as dCas9-ST-GFP-iLID) allowing for visualization of seeded sites, at the same time providing light inducible binding scaffolds for recruiting various protein components, including proteins that contain intrinsically disordered regions (IDRs).

IDRs from a number of nuclear proteins may be used to, e.g., examine phase behaviors. A score greater than 0.5 from the Predictor Of Natural Disordered Regions (PONDER) VL-XT algorithm may indicate a region is likely disordered. Transcriptional regulators including BRD4, TAF15 and FUS; BRD4 has received much recent attention, as it contains a long IDR (BRD4$\Delta$N), is highly enriched in enhancer clusters, and is thought to play an important role in transcriptional activation as well as elongation. However, because the disclosed system expresses these IDRs fused to the protein sspB, binding between iLID and sspB upon blue light activation leads to formation of IDR oligomers at the target genomic loci, which in turn drive localized liquid-liquid phase separation.

One example demonstrates the capacity of each CasDrop module targeted to telomeres, repetitive heterochromatic DNA elements found at the end of chromosomes. When dCas9-ST-sfGFP-iLID is expressed, several fluorescent puncta appear in the nucleus in a manner dependent on the expression of single guide RNA (sgRNA) cognate for telomeres. Light-induced phase separation of the CasDrop system in the absence of any sgRNA can then be examined. Prior to blue light activation, both dCas9-ST-GFP-iLID and IDR-mCh-sspB show diffuse fluorescent signals in the nucleus (similar to image seen in Fig. 2B). Upon blue light exposure, formation of IDR oligomers through dCas9-ST-GFP-iLID scaffolds leads to rapid clustering of liquid-like protein assemblies enriched with the CasDrop components; these assemblies are highly dynamic, reversible and undergo frequent droplet coalescence events. In control experiments without the presence of either dCas9-ST or IDR, no condensates are observed upon light activation, confirming the role of IDR oligomers as a driver for phase separation.

Locus-specific targeting with sgRNA can alter the way phase separation proceeds. When the CasDrop system is co-expressed with sgRNA for telomeres, bright GFP foci are observed to colocalize with telomeric repeat-binding factor TRF1, indicating successful scaffold targeting. Upon blue light activation, BRD4$\Delta$N-mCh-sspB liquid droplets nucleate and grow at the seeded telomeres. Interestingly, the number of droplets that are nucleated away from telomeres depends sensitively on the activation protocol: for a rapid increase in blue light, many droplets nucleate throughout the nucleoplasm, while for a light intensity ramping protocol, droplets nucleate almost exclusively at the telomere loci. This behavior is consistent with the classic physics of nucleation barriers for phase separation: for rapid activation, the system becomes deeply supersaturated in a short time, lowering the nucleation barrier to form many nuclei. For the incremental activation, on the other hand, initially the supersaturation level is low, allowing for preferential condensation at the seeded sites and then in later stages, existing droplets keep growing rather than forming costly new nuclei.

Simulations of the CasDrop system confirm this picture.

Control experiments without sgRNA for telomeres show that BRD4 droplets appear in an apparently random manner irrespective of telomeres. Collectively, these results indicate that droplet localization during intracellular phase separation can be dynamically controlled by pre-seeding and the rate of supersaturation. The CasDrop system thus functions to localize a nucleating scaffold, thereby driving phase separation at the genomic location defined by the sgRNA.

In studies of the off-target nucleation of CasDrops, droplets appear to form in regions of relatively low chromatin density. To quantify this, cells not expressing condensate-targeting guide RNA were examined to determine where droplets form relative to the chromatin distribution prior to droplet condensation, using H2B-miRFP670 as a proxy for chromatin density. It was found that the distribution of H2B intensity in regions where droplets do form is shifted to significantly lower H2B intensity compared with the H2B distribution in the entire nucleus. Dividing these two distributions reveals that the propensity for droplet formation is a strong function of normalized H2B intensity, with droplets exhibiting a significant preference for regions of low chromatin density. Notably, the observed trend is not due merely to variations in the concentrations of CasDrop components, as similar analysis does not show biased distributions of the components.

The chromatin density was examined after droplets had formed. Remarkably, as the droplets grow, chromatin is significantly pushed out, creating easily visualized "holes".

Chromatin exclusion is not an artifact of optogenetic scaffolding, as we see similar behavior for condensates formed with a variety of YFP-tagged IDR-containing full-length proteins overexpressed in cells. These findings are consistent with the observation that several endogenous nuclear condensates, including nucleoli, Cajal bodies (CBs), PML bodies, nuclear speckles, and paraspeckles are also associated with particularly low-density chromatin. Thus, condensates assembled from IDRs exhibiting a broad spectrum of sequence features (see Table 1, below) not only preferentially nucleate in low density chromatin regions, but upon nucleation and growth they also physically exclude chromatin.

Table 1

| Gene Name | Function | FCR | NCPR | Hydropathy | Disorder | Domains/Motifs (Uniprot) |
|---|---|---|---|---|---|---|
| CCNT1 | P-TEFb complex, transcription elongation | 0.213 | 0.012 | 3.798 | 0.697 | Coiled-coil |
| HSF1 | Stress-inducible transcription factor | 0.216 | -0.042 | 4.113 | 0.681 | TAD, DNA-binding domain |
| MLLT3 | Super elongation complex, transcription | 0.305 | 0.012 | 3.447 | 0.739 | YEATS |
| RNPS1 | Splicing, exon junction comlex | 0.354 | 0.177 | 2.931 | 0.849 | RRM |
| SART1 | mRNA processing and splicing | 0.385 | -0.013 | 3.416 | 0.751 | Coiled-coil (2) |
| TAF15 | Splicing, RNA binding, transcription | 0.24 | 0.003 | 2.978 | 0.792 | Zinc-finger, RRM, RGG box |

To gain physical insight into why droplets that tend to exclude chromatin would preferentially grow in regions of low chromatin density, the mechanical interplay of condensates with the deformable chromatin network can be modeled. By excluding chromatin, growing droplets give rise to mechanical stresses; indeed, in non-biological systems, phase separated droplets are known to be strongly impacted by the presence of a surrounding elastic network. A minimal model can be used to describe droplet formation in chromatin as an expanding sphere creating a cavity in an elastic matrix.

The following relation between pressure and size for a spherical cavity was considered, derived from a neo-Hookean strain energy relation to determine the inward pressure on a cavity with deformation ratio $\lambda$ in an incompressible elastic medium of Young's modulus $G$:

$$\frac{P}{G} = \frac{5}{6} - \frac{2}{3\lambda} - \frac{1}{6\lambda^4}. \qquad \text{(Eq. 1)}$$

This pressure, which reflects the energetic cost of deforming the chromatin, complements classical nucleation theory to describe the energetics of droplet formation in a dense elastic matrix. The energy cost of deforming the elastic chromatin matrix is then added to the contributions of bulk chemical potential gain and surface tension cost to obtain the total free energy cost to create a spherical droplet of radius $R$, obtaining:

$$\Delta F(R) = 4\pi R^2 \gamma - \frac{4}{3}\pi R^3 \left(\Delta\mu \cdot c_{drop} - \frac{5}{6}G - \frac{2}{3\lambda} - \frac{1}{6\lambda^4}\right), \qquad \text{(Eq. 2)}$$

where $\gamma$ is the surface tension of the droplet, $\Delta\mu$ is the chemical potential difference between molecules in the supersaturated solution and the droplet phase, $c_{drop}$ is the saturated bulk concentration of molecules inside the droplet, $\lambda = R/r_{mesh}$, and $r_{mesh}$ is a typical local mesh size in the chromatin network. Since $r_{mesh} \ll R$, the deformation ratio $\lambda$ is very large. Thus, the following simplified expression for the free energy cost $\Delta F$ of droplet nucleation is given by:

$$\Delta F(R) = 4\pi R^2 \gamma - \frac{4}{3}\pi R^3 \left(\Delta\mu \cdot c_{drop} - \frac{5}{6}G\right). \qquad \text{(Eq. 3)}$$

where $R$ is the radius of the droplet, $\gamma$ is the surface tension of the droplet, $\Delta\mu$ is the chemical potential difference between molecules in the supersaturated solution and in the droplet phase, $c_{\text{drop}}$ is the saturated bulk concentration of molecules inside the droplet, and $G$ is the elastic (Young's) modulus of the deformed surrounding matrix. The first two terms reflect classical nucleation theory, while the third term reflects a mechanical energy contribution from the chromatin elasticity.

For values of $\Delta\mu \cdot c$drop less than the critical pressure $P_c$ = 5G/6, the free energy increases to infinity for large $R$, suggesting that for sufficiently dense chromatin, droplet size is restricted. However, when $\Delta\mu \cdot c_{drop} > P_c$, the droplet can grow without constraint. If the elastic environment is heterogeneous, as chromatin density is in the cell, then regions of low stiffness should be favored for phase separation and out-compete denser regions for molecules.

Various parameters were estimated for use with this model, based on literature.

$\gamma \approx 4 \times 10^{-7}$ N/m for nucleolar protein in vitro.

$r_{mesh} \approx 7$ - 13 nm. A rough estimate of pore size can be given by the mean free path between chromatin fibers

$$r_c \sqrt[a]{\frac{1-f}{f}}$$

based on the chromatin size and volume fraction: $r_{\text{mesh}} =$ where $r_c$ is the width of a chromatin fiber, estimated at 7 nm and $f$ is the volume fraction of chromatin. The fraction has been estimated by electron microscopy at approximately 0.12-0.21 in euchromatin and about 0.37-0.52 for heterochromatin, giving pore sizes of about 14 nm and 7 nm respectively.

$c_{\text{drop}}$ has been estimated using fluorescence correlation spectroscopy both in the similar biomimetic Corelet system and for in vitro droplets to be approximately 5-10 $\times$ 10$^{-5}$ molecules/nm$^3$.

$\Delta\mu$ is estimated at approximately 2-5$k_B T$, being the chemical potential difference per molecule between the dilute and condensed phases.

$G$ is dependent on the crosslink frequency of the matrix, which can be estimated by $f^2$, the square of the volume fraction of the matrix. The effective "spring constant" of the nucleus for a lamin knockout has been estimated to be on order 1 nN/$\mu$m, which dividing by a microscopic length scale of 1 $\mu$m would give about 1 kPa for the nucleus on average. Based on this, it can be assumed that the least stiff chromatin, with a fraction of about 0.21 has a modulus of about 100 Pa and that this value scales with $f^2$.

The minimal mathematical model for condensate thermodynamics within an elastic medium was implemented within a diffuse-interface formalism that permits numerical study of collective droplet nucleation, growth, and coarsening kinetics with mechanical effects. In one example, the nucleoplasmic fluid is described as an effectively ternary system composed of dCas9-ST + scFV-sfGFP-iLID (species A), TR-mCh-ssp (species B; TR=Transcriptional Regulator), and other "solvent" molecules (species C). An extended ternary regular solution free energy functional is employed to describe fluid thermodynamic phase behavior within a mechanical network;

$$F = \int \left[ \phi_A \ln \phi_A + \phi_B \ln \phi_B + \phi_C \ln \phi_C + X_{AB}\phi_A\phi_B + X_{AC}\phi_A\phi_C + X_{BC}\phi_B\phi_C \right.$$
$$\left. + \sum_i \left[ \tilde{\lambda}_i(\vec{r}, R)\nabla\phi_i \right] + 5G(\vec{r})(\phi_A + \phi_B)/6 + P_A(\vec{r})(1 - \phi_A)^2 \right] d\vec{r}$$

where $\phi_i$ is the space-dependent volume fraction of molecular population $i \in \{A, B, C\}$; $X_{ij}$ controls the strength of interaction between $i$ and $j$ molecules; $\tilde{\lambda}_i(\vec{r}, R)$ is the network-modified surface energy coefficient for population $i$ (see below); $G(\vec{r})$ is the space-dependent Young's modulus of the network; and $P_A(\vec{r})$ is a field that enhances the concentration of species A at pre-seed sites. The fluid phase is taken to be incompressible such that $\phi_A + \phi_B + \phi_C = 1$. Dynamics are given by a generalized diffusion equation, $\dfrac{\partial \phi_i}{\partial t} = M_i \nabla^2 \dfrac{\partial F}{\partial \phi_i}$, where $i, j \in \{A, B\}$ ( C is eliminated via incompressibility), $M_i$ is the mobility of population $i$, and $t$ is dimensionless time.

The effect of the elastic medium is incorporated into a space-dependent bulk term (that proportional to $G(\vec{r})$) which locally modulates the chemical potentials, and a space- and droplet size-dependent interfacial energy coefficient

$\lambda_i(\vec{r},R) = \lambda_i + \dfrac{G(\vec{r})r_{mesh}\left(2 + \frac{r_{mesh}^3}{2R}\right)/9}{}$ ) where $\lambda_i$ is the surface energy coefficient in the absence of a network. The interfacial terms associated with the network are negligible compared to the bulk network term when R $\gtrsim 10r_{\text{mesh}}$. One can therefore neglect the effect of the network on interfacial energy (let $\tilde{\lambda}_i \approx \lambda_i$) and study the regime in which $R \gtrsim$

$10r_{mesh}$.

Telomere pre-seeding simulations were conducted by initializing a homogeneously mixed fluid with $\phi_A$= 0.1, $\phi_B$= 0.1, $\phi_C$ = 0.8, $X_{AC}=X_{BC}$=1, $\lambda_i$ = 0.75, and setting $G(\vec{r})$ = 0.6 within randomly positioned pre-seed regions, $P_A(\vec{r})$ = 0.005 within an annulus of radius 10 around each stiff pre-seed core, and $G(\vec{r})$ = $P_A(\vec{r})$ = 0 elsewhere. Nonzero $P_A$ values enhance the local concentration of A around pre-seed sites, analogous to the initial enhancement of dCas9-ST + scFV-sfGFP-iLID around telomeres in the experimental system. Blue light-induced heterodimerization of sspB with iLID is described as an increase in A-B interaction strength, $X_{AB} = X_{AB}^0 + (X_{AB}^{max} - X_{AB}^0)(1 - e^{-t/\tau[blue]})$, where $X_{AB}^0 = -5$ and $X_{AB}^{max} = -9.25$ are the inactivated state and activated state interaction strengths, respectively. $\tau$[blue] is the time constant for blue light activation, which is inversely proportional to the rate of increase in blue light intensity. Decreasing $X_{AB}$ from -5 to -9.25 induces phase separation into A+B-rich droplets and an A+B-poor background.

Following equilibration with pre-seed sites as described above, blue light is applied globally with intensity increasing according to the equation above for a given value of $\tau$[blue]. Droplets are more effectively localized at pre-seed sites with decreasing quench rate. The simulations demonstrate that enhanced concentration of A at pre-seed sites promotes rapid local nucleation and subsequent diffusion-limited growth. Growth proceeds by drawing in nearby A and B molecules, which creates an expanding radial zone depleted in A and B. If depletion zones of neighboring pre-seed sites overlap before droplets can nucleate between pre-seeds, then all droplets become localized at pre-seed sites. If droplets nucleate throughout the system before depletion zones overlap, then long-lived droplets also appear away from seed sites. For ideal diffusion-limited growth, the radius of the depletion zone grows as $R_{DZ} \approx \sqrt{2DS_0 t}$, where $D$ is the diffusion coefficient of A and B molecules and $S_0$ is their supersaturation. The time required for overlap of neighboring depletion zones is therefore $t^* \approx \left(\frac{d}{2}\right)^2 /2DS_0$, where $d$ is the distance between pre-seed sites. The simulations with a spatially heterogeneous elastic network were conducted with blue light applied globally as described above, but with $P_A(\vec{r})$ = 0 and $G(\vec{r})$ = $G_0$[cos($4\pi x/L_x$) + cos($4\pi y/L_y$) + 2]/4, where $L_x$ and $L_y$ are the lengths of the simulation cell. The constant $G_0$ was increased linearly from 0 to 0.18 between $t$ = 250 (after droplet nucleation) and $t$ = 750. This delay in the introduction of mechanical deformation energy results in spatially uniform droplet nucleation and an initial droplet size distribution that is independent of network stiffness, consistent with our neo-Hookean model. The subsequent introduction of nonzero $G(\vec{r})$ induces a transition into the large droplet size regime discussed above, in which the dominant effect is a shifting of the bulk chemical potential according to local stiffness and preferential droplet growth in softer regions. Additional simulations were conduct as described above, but with blue light applied locally inside a stiff hete-rochromatin-like domain, wherein $G_0$ was increased linearly from 0 to 0.18 between $t$ = 100 (after droplet nucleation) and $t$ = 600.

When two droplets seeded at specific genomic loci fuse, surface tension favors making the resulting large droplet spherical. This results in a force that attracts the two loci towards one another, displacing each one a distance $\Delta x$ from its original position. This displacement induces deformation in the chromatin that results in an elastic restoring force pulling the loci back towards their original positions; at mechanical equilibrium, the balance between droplet surface area and loci displacement reflects the relative magnitude of the surface tension $\gamma$ and chromatin Young's modulus $G$. Quantitatively, when two droplets of radius $R$ fuse to form a sphere, the resulting droplet has an equilibrium radius of $2^{1/3}R$. Due to surface tension, there is an energetic cost to elongating the droplet from the spherical configuration. This results in a force $F_{tension}$ between the telomeres, which to linear order in the elongation $\delta = 2R - 2^{\frac{1}{3}}R - \Delta x$, and assuming that the droplet is constrained to remain ellipsoidal, reads $F_{tension} = \gamma \left( 2R - 2^{\frac{1}{3}}R - \Delta x \right)$. The elastic force on a locus of size $r_{locus}$ displaced by $\Delta x$ is given by the elastic Stokes' law, $F_{elastic}$ = -6$\pi Gr_{locus} \Delta x$. Force balance thus yields

$$\Delta x \approx \gamma \left( 2R - 2^{\frac{1}{3}}R \right) /(y + 6\pi Gr_{locus}). \qquad \text{(Eq. 4)}$$

Taking a telomere size of about 10 nm, a $\gamma$ of 4 × 10$^{-7}$ N/m as before, a droplet size of 1 $\mu$m and a $G$ in the range of 10-100 Pa results in a displacement $\Delta x \approx$ 10-100 nm.

Thus, a prediction of the model is that mechanical deformation energy will prevent droplets from growing beyond a critical size in sufficiently dense heterochromatic regions, i.e., those near the high-density end of the experimentally-measured droplet growth propensity. To test this prediction, major satellite repeats, dense heterochromatin regions prominent in mouse cultured cells which are highly enriched in the heterochromatin protein HP1$\alpha$. The CasDrop system is then used to locally assemble BRD4 condensates by focusing the laser within the nucleus. When BRD4 droplets are induced in euchromatin regions, devoid of HP1$\alpha$-miRFP670 label, BRD4 droplets readily condense. However, when attempting to write droplets directly onto heterochromatin, resolvable droplets tend to condense only around the periphery; this effect is not a result of IDR exclusion, since it robustly occurs even after enrichment of IDR-sspB component through sgRNA targeting. Simulations of condensate nucleation at a compact heterochromatin core demonstrate flower petal-like configurations strikingly similar to those seen in these experiments. Interestingly, in some cases HP1$\alpha$-miRFP670 enriched heterochromatin foci exhibit apparent subregions of weak HP1$\alpha$-miRFP670 fluorescence, which may reflect some degree of interspersed euchromatin. When attempting to nucleate BRD4 droplets on these foci, BRD4 droplets occasionally nucleate within; as they grow, they displace HP1$\alpha$, as seen from the anti-correlated fluorescence intensities; strikingly, the growing BRD4 droplets can even "rupture" heterochromatin, abruptly spilling out into euchromatic regions. We observe qualitatively similar heterochromatin immiscibility with TAF15 and FUS CasDrops. Collectively, these behaviors provide strong support for the presence of mechanical stresses that build up in chromatin surrounding growing condensates. These data are thus consistent with our mechanical droplet exclusion model, although HP1$\alpha$ itself could provide additional unfavorable interactions to reinforce this mechanical immiscibility.

These findings of mechanical chromatin exclusion are surprising, given that enhancer clusters and other nuclear condensates have been hypothesized to bring specifically-targeted genomic elements into closer proximity. To explore these mechanical effects, the condensates targeted to telomeres were examined. Consistent with the findings above, BRD4 CasDrop condensates do not appear to mix with the targeted heterochromatic telomeres, which tend to localize at the droplet periphery. But the two nevertheless partially wet one another, and are thus adherent. When two droplets seeded at different telomeres combine with one another, the surface tension is sufficient to produce correlated motion between loci, and pull them into close proximity. Ignoring viscous effects, telomere displacement should be given by the ratio of droplet surface tension, $\gamma$, and effective genome elasticity, $G$, i.e.,

$\Delta x \sim \gamma/G$; estimated values for these two parameters yield predicted displacements that are consistent with these measurements. In some cases, droplets can detach from associated telomeres, which then relax back to a more distal position, consistent with droplet detachment releasing the intervening "spring-loaded" chromatin.

Exemplary Systems

Methods Cell culture.

NIH3T3, HEK293, HEK293T, and U2OS cells were cultured in growth medium consisting of Dulbecco's modified Eagle's medium (Gibco), 10% fetal bovine serum (Atlanta Biologicals), and 10 U/mL Penicillin-Streptomycin (Gibco) and incubated at 37°C and 5% $CO_2$ in a humidified incubator.

Transient transfection.

HEK293, HEK293T, or U2OS cells were grown to approximately 70% confluency in 12-well plates before being transfected with plasmid DNA using Lipofectamine 3000 (Invitrogen) following manufacturer's protocol. Briefly, transfection reagents and DNA plasmids were diluted with OPTI-MEM (Gibco). Each well received 100 $\mu$L of transfection mixture containing a total of 1 $\mu$g DNA. The transfection mixture was removed 6-24 hours post transfection. Cells transiently transfected were typically imaged between 24-48 hours post transfection.

Lentiviral transduction.

Lentivirus was produced by cotransfecting the transfer plasmids, pCMV-dR8.91, and pMD2.G (9:8:1, mass ratio) into HEK293T cells grown to approximately 70% confluency in 6-well plates using FuGENE HD Transfection Reagent (Promega) per manufacturer's protocol. A total of 3 $\mu$g plasmid and 9 $\mu$L of transfection reagent were delivered into each well. After 2 days, supernatant containing viral particles was harvested and filtered with 0.45 $\mu$m filter (Pall Life Sciences). Supernatant was immediately used for transduction, concentrated 10-fold using Lenti-X Concentrator (Takara), or stored at -80°C in aliquots. NIH3T3 or HEK293T cells were grown to 10-20% confluency in 12-well plates and 100-1000 $\mu$L of filtered viral supernatant was added to the cells. Media containing virus was replaced with fresh growth medium 24 hours post-infection. Cells infected were typically imaged no earlier than 72 hours after infection.

Cell line generation.

To establish cell lines expressing multiple constructs, sequential lentiviral transduction was performed, together with fluorescence activated cell sorting (FACS) when needed. Wild-type NIH3T3 (or HEK293T) cells were transduced with lentivirus containing dCas9-ST under SFFV promoter and scFv-sfGFP-iLID. This transduced NIH3T3 cell line was then used to generate other cell lines by lentiviral transduction, to express required constructs indicated for each experiment. To increase population expressing dCas9-ST in the transduced HEK293T cell line, cells were sorted on a FACSAria Fusion flow cytometer (BD Biosciences) with gating for single-cells expressing high level of BFP and intermediate level of GFP. Polyclonal cell pool was collected, grown and recovered in growth medium. This sorted cell line was then transiently transfected with additional constructs for CasDrop/Cry2-fusion experiment.

Constructs.

FUSN-mCh-sspB was first generated by inserting FUSN (1-214), mCherry, and sspB coding sequence into a pHR-based vector (Shin et al., 2017). For other TR-mCh-sspB constructs, the FUSN in FUSN-mCh-sspB was swapped out for the DNA sequence encoding BRD4ΔN (462-1362), TAF15N (1-208). scFv-sfGFP-iLID was generated by adding iLID (Addgene 60413) between GB1 and NLS in scFv-GCN4-GFP (Addgene 60906). A promoter for dCas9-ST (Addgene 60910) was modified from dSV40 to SFFV to enhance expression. Fragments of mCherry, sspB (Addgene 60415), miRFP670 (Addgene 79987), TRF1 (Addgene 64164) and HP1α (Addgene 17652) were amplified by PCR. All Cry2 fragments used were identical to the one described previously (Shin 515 et al., 2017) except for a fluorescent reporter swapped from mCh to miRFP670. For EYFP constructs, FM5-EYFP (kind gift from Marc Diamond lab, UT Southwestern) was digested with NheI and open-reading frames were subcloned onto 5' end using In-Fusion Cloning Kit (Takara). To create FMS-ORF-mCherry-Cry2 constructs, FM5-EYFP was digested with NheI and AscI and DNA backbone lacking EYFP was gel purified using Qiagen Gel Extraction Kit. mCherry-Cry2 was PCRed from pHr-mCherry-Cry2, digested with NheI and AscI, gel purified, and ligated into FM5 backbone using Quick Ligase (NEB). FM5-mCherry-Cry2 was then digested with NheI and open reading frames were subcloned onto 5' end using In-Fusion. All open reading frames were PCRed from recombinant DNA vectors obtained from AddGene: CCNT1 (14607), HSF1 (32538), MI,LT3 (49428), SART1 (38087), TAF15 (84896), BMI1 (69796), SRSF2 (84020), PRPF6 (51740), with the exception of RNPS1, which was synthesized by GenScript. For sgRNAs targeting telomeres and major satellite repeats (sgTel, sgMaj), pLV-sgCDKN1B (Addgene 60905) was first digested with BstXI and XhoI followed by gel electrophoresis and extraction. Then, PCR fragments for sgRNAs were generated using a sequence-specific forward primer and a common reverse primer. All fragments were ligated and assembled into the final vectors using In-Fusion Cloning Kit (Takara).

Immunocytochemistry.

HEK293 cells expressing H2B-miRFP670 were fixed using 3.5% PFA (Electron Microscopy Services) in PBS for 15 minutes. Cells were washed twice with PBS and permeabilized with 0.25% Triton-X in PBS for 20 minutes. Non-specific epitopes were blocked for one hour using blocking buffer (PBS, 0.1% Triton-X, 10% normal goat serum from Vector Laboratories). Primary immunostaining was performed with the following antibodies overnight at 4°C in blocking buffer: PML (Mouse, AbCam ab11826, 1 to 50), Coilin (Rabbit, Santa Cruz sc32860, 1 to 100), TDP43 (Rabbit, Protein-Tech 10782-2-AP, 1 to 100), SMN1 (Mouse, Santa Cruz sc-32313, 1 to 100), SC35 (Mouse, AbCam ab11826, 1 to 1000), and FBL (Mouse, AbCam ab4566, 1 to 40). Cells were then washed 3X with 0.1% Triton-X in PBS. Secondary immunostaining was performed with the following antibodies from Invitrogen in blocking buffer at room temperature for 90 minutes: AlexaFluor 546 goat anti-rabbit (A11010, 1 to 400), AlexaFluor 546 goat anti-mouse (A11030, 1 to 400). Cells were washed 3X with 0.1% Triton-X in PBS. DNA was visualized with 2 μg/mL Hoechst dye (ThermoScientific), staining for 15 minutes in PBS. Finally, Hoechst was removed and replaced with PBS prior to imaging. Controls without primary antibodies were performed to ensure specificity of primary stain.

Microscopy.

All images are taken using 60X immersion objective (NA 1.4) on a Nikon A1 laser scanning confocal microscope. An imaging chamber is maintained at 37°C and 5% CO2. For live cell imaging, cells are plated on the fibronectin (Sigma-Aldrich) coated 35-mm glass-bottom dishes (MatTek) and grown typically overnight. For global activation, cells are usually imaged with a 488-nm laser but when the blue light intensity needs to be reduced due to high sensitivity of the optogenetic proteins (iLID and Cry2), a 440-nm laser is used in conjunction with a dichroic filter for the 488-nm laser. This allows for attenuation of the blue laser intensity at the specimen plane below 0.1 μW. For local activation, a region of interest (ROI) is defined to guide area to be scanned with blue lasers. Fluorescence recovery after photobleaching (FRAP) is performed similarly using ROI.

Image analysis.

All data analysis on images was performed using custom-built MATLAB scripts. Briefly, for telomere tracking, raw images were first Gaussian filtered to reduce noise and then peaks corresponding to telomeres are detected based on their peak intensity. Trajectories are generated from a series of detected coordinates based on proximity. To identify and track the boundary of either droplets or heterochromatin, segmented binary images are obtained using the edge detection routine in MATLAB. Analyzed results are manually inspected to check validity.

The disclosed shows how nuclear condensates can both sense and restructure their local genomic environment. A broad range of different IDR-containing proteins exclude chromatin, which in some cases manifests in large scale deformation of the chromatin network. These proteins display a remarkable diversity of their physical characteristics, exhibiting IDRs that range from relatively uncharged (e.g. TAF15 N) to highly basic (e.g. SRSF2 IDR) and mixed-charge (SART1); from relatively hydrophobic (e.g. HSF1) to highly hydrophilic (e.g. RNPS1). Thus, mechanical exclusion of chromatin appears to occur irrespective of the physicochemical properties of the protein that drives phase separation, and likely underlies the low chromatin density found within various IDR-rich nuclear bodies. These findings are consistent with the observation in a prior study that the germ plasm protein DDX4 appears to exclude chromatin. Interestingly, DDX4 condensates do not exclude single stranded RNA and DNA, which instead strongly partition into the droplets. Such selectivity could potentially help facilitate the flow of genetic information, as the single-stranded RNA transcripts from excluded chromatin are pulled into adjacent condensates.

The tendency for nuclear condensates to exclude chromatin has dramatic consequences within the heterogeneous nuclear environment. Exclusion of chromatin from a growing droplet causes the chromatin network to be deformed. Deformation of elastic (or viscoelastic) materials gives rise to a strain energy, which represents a mechanical energy stored within the matrix . This deformation thus represents an energetic cost that is thermodynamically unfavorable, such that the elastic properties of the matrix become an important factor in the growth dynamics of the droplet. In non-living systems, it is known that this effect can strongly impact phase separation, and can even give rise to uniform droplets of a size set by the matrix elasticity. The theoretical analysis and simulations show that as a result of this deformation energy, droplets will tend to favor growth in softer, lower density regions of the genome. Small droplets that do form in heterochromatin ultimately dissolve and act as IDR sources for the growing droplets within the softer euchromatin regions. The striking consequences of this effect can be observed, in flower-petal-like arrangement of droplets around heterochromatin foci, or in rare cases the extrusion of droplets from within dense and mechanically stiffer heterochromatin. This effect can thus give rise to genomic rearrangements, and is likely important for promoting preferential growth of transcriptional condensates in mechanically softer, low-density genomic regions associated with active gene expression.

The method and system also shed light on how targeted condensates can bring distant loci together. Many IDR-rich proteins possess targeting "reader" motifs, such as the bromodomain found in BRD4, which targets this phase separation-prone protein to histones exhibiting acetylated lysine resides. The CasDrop system replaces such endogenous targeting motifs with programmable dCas9, enabling dissection of the biophysical consequences of IDP targeting. We use CasDrop to show how IDP targeting promotes localized phase separation, a process which appears closely related to the "diffusive capture" mechanism, which can amplify IDR concentration to drive localized phase separation. Our work also reveals how surface-tension can mediate targeted droplet coalescence. We quantify the associated forces and genomic deformations, which can bring two or more targeted loci into closer proximity. This ability to "pull in" targeted genomic loci, can be contrasted with our finding that a broad spectrum of IDR-driven condensates does the opposite to non-targeted genomic elements, i.e. "pushing them out". Combing these findings, we propose a chromatin filter model for condensate-induced genome restructuring, in which transcriptionally active condensates such as the nucleolus, and super-enhancer clusters, play bifunctional roles, serving to both filter out non-specific elements of the genome, while pulling together targeted regions to which they are bound.

## Claims

1.  A system for the controlled clustering of gene regulatory proteins at specific target genomic loci, comprising:

> a first structure comprising;

>> (i) a first segment having a Cas-based genomic targeting protein fused or attached to one or more first sequences, each first sequence including at least one sequence selected from the group consisting of a light-sensitive receptor, a chemical-sensitive receptor, and a light-sensitive oligomerization protein; or
>> (ii) a first segment having a Cas-based genomic targeting protein fused or attached to one or more genes encoding at least one protein sensitive to at least one wavelength of light and a second segment fused to the first segment, the second segment having at least one sequence selected from the group consisting of a low complexity sequence (LCS), an intrinsically disordered protein region (IDR), a synthetic or natural nucleic acid binding domain, and at least one repeatable sequence, wherein the repeatable sequence comprises a linker fused to at least one additional gene encoding at least one protein sensitive to at least one wavelength of light; and

a second structure comprising a second sequence selected from the group consisting of a cognate partner of the light-sensitive receptor protein, a cognate partner of the chemical-sensitive protein, and a dimerization domain complementary to a dimerization module, the second sequence being fused to at least one transcriptional regulatory protein having a full length or truncated low complexity or intrinsically disordered protein region, wherein the protein system is configured for targeted condensation at the specific target genomic loci by exposing the protein system to at least one predetermined wavelength of light, a chemical to which the chemical-sensitive receptor is sensitive, or a combination thereof.

2. The system according to claim 1, wherein the first structure includes a first construct comprising dCas9 fused to SunTag attached to a second construct comprising a single chain variable fragment antibody fused to a superfolding variant of GFP (sfGFP) and iLID, where the single chain variable fragment antibody is a cognate for SunTag.

3. The system according to any one of claims 1 or 2, wherein the first structure comprises at least one reporter protein, the second sequence is fused to full length or truncated BRD4, FUS, or TAF15, or any combination thereof.

4. The system according to any one of claims 1-3, wherein the one or more first sequences includes a repeating first sequence.

5. An in vitro method for the controlled clustering of gene regulatory proteins at the specific target genomic loci, comprising the steps of:

(a) providing at least one specific genomic target loci and complementary single-guide RNA;
(b) providing a protein system in a living cell, the system including either:

(i) a first segment having a Cas-based genomic targeting protein fused or attached to one or more sequences, each sequence including a light-sensitive receptor, chemical-sensitive receptor, oligomerization protein, or a combination thereof; or
(ii) a first segment having a Cas-based genomic targeting protein fused or attached to at least one gene encoding at least one protein sensitive to at least one wavelength of light; and a second segment fused to the first segment, the second segment having at least one sequence selected from the group consisting of a low complexity sequence (LCS); an intrinsically disordered protein region (IDR); a synthetic or natural nucleic acid binding domain; and at least one repeatable sequence, the repeatable sequence comprising a linker fused to at least one additional gene encoding at least one protein sensitive to at least one wavelength of light; and

(c) inducing targeted condensation at the specific target genomic loci by exposing the protein system to at least one predetermined wavelength of light, a chemical to which the chemical-sensitive receptor is sensitive, or a combination thereof.

6. The method according to claim 5, wherein the system further comprises a second segment having a cognate partner of the light- or chemical-sensitive receptor protein fused to at least one gene regulatory protein having a full length or truncated low complexity or intrinsically-disordered protein region, or other folded proteins known to promote self-interactions.

7. The method according to claim 5, further comprising sensing or measuring the targeted condensation to detect the mechanical state of the genome.

8. The method according to claim 7, wherein detecting the mechanical state of the genome includes detecting the presence of tightly condensed heterochromatin.

9. The method according to any of claims 5-8, wherein inducing targeted condensation at the specific target genomic loci causes at least one change selected from the group consisting of inducing mechanical stresses in the genome, relaxing mechanical stresses in the genome, and altering mechanical stresses in the genome.

10. The method according to any one of claims 5-9, wherein inducing targeted condensation includes inducing enhancer-promoter interactions, inducing super enhancer clusters, actuating related types of proximity-dependent gene regulatory elements, inducing heterochromatization, or a combination thereof.

11. The method according to any one of claims 5-10, wherein the system includes a first protein system associated with a first genomic locus, and a second protein system associated with a second genomic locus, and wherein exposing the first and second protein systems to the at least one wavelength of light causes droplets to form, then coalesce together.

12. The method according to claim 11, wherein when the droplets are formed, the center-to-center separation of the two droplets is less than 3 microns.

13. The method according to any one of claims 5-12, wherein the first structure includes a first construct comprising dCas9 fused to SunTag attached to a second construct comprising a single chain variable fragment antibody fused to a superfolding variant of GFP (sfGFP) and iLID where the single chain variable fragment antibody is a cognate for Sun Tag.

14. The method according to any one of claims 5-13, further comprising allowing the first structure to serve as a multimeric protein scaffold for binding of the second structure; and triggering liquid-liquid phase separation by exposing the first structure to at least one predetermined wavelength of light and causing the first and second structure self-assemble into a multimeric protein complex.

**Patentansprüche**

1. System zum gesteuerten Clustern von genregulatorischen Proteinen an spezifischen genomischen Target-Loci, umfassend:

eine erste Struktur, umfassend;

(i) ein erstes Segment, das ein Cas-basiertes genomisches Targeting-Protein aufweist, das an eine oder mehrere erste Sequenzen fusioniert oder gebunden ist, wobei jede erste Sequenz mindestens eine Sequenz enthält, die aus der Gruppe ausgewählt ist, die aus einem lichtempfindlichen Rezeptor, einem chemikalienempfindlichen Rezeptor und einem lichtempfindlichen Oligomerisierungsprotein besteht; oder
(ii) ein erstes Segment, das ein Cas-basiertes genomisches Targeting-Protein aufweist, das an ein oder mehrere Gene fusioniert oder gebunden ist, die für mindestens ein Protein codieren, das für mindestens eine Lichtwellenlänge empfindlich ist, und ein zweites Segment, das an das erste Segment fusioniert ist, wobei das zweite Segment mindestens eine Sequenz aufweist, die aus der Gruppe ausgewählt ist, die aus einer Sequenz mit geringer Komplexität (LCS), einer intrinsisch ungeordneten Proteinregion (IDR), einer synthetischen oder natürlichen Nukleinsäurebindungsdomäne und mindestens einer wiederholbaren Sequenz besteht, wobei die wiederholbare Sequenz einen Linker umfasst, der an mindestens ein zusätzliches Gen fusioniert ist, das für mindestens ein Protein codiert, das für mindestens eine Lichtwellenlänge empfindlich ist; und

eine zweite Struktur, die eine zweite Sequenz umfasst, die aus der Gruppe ausgewählt ist, die aus einem kognaten Partner des lichtempfindlichen Rezeptorproteins, einem kognaten Partner des chemikalienempfindlichen Proteins und einer zu einem Dimerisierungsmodul komplementären Dimerisierungsdomäne besteht, wobei die zweite Sequenz an mindestens ein transkriptionelles regulatorisches Protein fusioniert ist, das eine Proteinregion mit geringer Komplexität oder eine intrinsisch ungeordnete Proteinregion entweder in voller Länge oder trunkiert aufweist,
wobei das Proteinsystem für eine gerichtete Kondensation an den spezifischen genomischen Target-Loci konfiguriert ist, indem das Proteinsystem mindestens einer vorbestimmten Lichtwellenlänge, einer Chemikalie, für die der chemikalienempfindliche Rezeptor empfindlich ist, oder einer Kombination davon ausgesetzt wird.

2. System nach Anspruch 1, wobei die erste Struktur ein erstes Konstrukt beinhaltet, das an SunTag fusioniertes dCas9 umfasst, das an ein zweites Konstrukt gebunden ist, das einen Antikörper mit einkettigem variablem Fragment umfasst, der an eine Superfaltungsvariante von GFP (sfGFP) und iLID fusioniert ist, wobei der Antikörper mit einkettigem variablem Fragment ein Kognat für SunTag ist.

3. System nach einem der Ansprüche 1 oder 2, wobei die erste Struktur mindestens ein Reporterprotein umfasst, die zweite Sequenz an BRD4, FUS oder TAF15 voller Länge oder trunkiert fusioniert ist, oder eine beliebige Kombination davon.

4. System nach einem der Ansprüche 1-3, wobei die eine oder mehreren ersten Sequenzen eine sich wiederholende erste Sequenz beinhalten.

5. In vitro-Verfahren zum gesteuerten Clustering von genregulatorischen Proteinen an den spezifischen genomischen Target-Loci, umfassend die folgenden Schritte:

(a) Bereitstellen von mindestens einem spezifischen genomischen Target-Locus und komplementärer Single-Guide-RNA;
(b) Bereitstellen eines Proteinsystems in einer lebenden Zelle, wobei das System entweder beinhaltet:

(i) ein erstes Segment, das ein Cas-basiertes genomisches Targeting-Protein aufweist, das an eine oder mehrere Sequenzen fusioniert oder daran gebunden ist, wobei jede Sequenz einen lichtempfindlichen Rezeptor, einen chemikalienempfindlichen Rezeptor, ein Oligomerisierungsprotein oder eine Kombination davon beinhaltet; oder
(ii) ein erstes Segment, das ein Cas-basiertes genomisches Targeting-Protein aufweist, das an mindestens ein Gen fusioniert oder gebunden ist, das für mindestens ein Protein codiert, das für mindestens eine Lichtwellenlänge empfindlich ist; und ein zweites Segment, das an das erste Segment fusioniert ist, wobei das zweite Segment mindestens eine Sequenz aufweist, die aus der Gruppe ausgewählt ist, die aus einer Sequenz mit geringer Komplexität (LCS), einer intrinsisch ungeordneten Proteinregion (IDR); einer synthetischen oder natürlichen Nukleinsäurebindungsdomäne; und mindestens einer wiederholbaren Sequenz besteht, wobei die wiederholbare Sequenz einen Linker umfasst, der an mindestens ein zusätzliches Gen fusioniert ist, das für mindestens ein Protein codiert, das für mindestens eine Lichtwellenlänge empfindlich ist; und

(c) Induzieren einer gezielten Kondensation an den spezifischen genomischen Target-Loci, indem das Proteinsystem mindestens einer vorbestimmten Lichtwellenlänge, einer Chemikalie, für die der chemikalienempfindliche Rezeptor empfindlich ist, oder einer Kombination davon ausgesetzt wird.

6. Verfahren nach Anspruch 5, wobei das System ferner ein zweites Segment umfasst, das einen kognaten Partner des licht- oder chemikalienempfindlichen Rezeptorproteins aufweist, der an mindestens ein genregulatorisches Protein fusioniert ist, das eine Proteinregion mit geringer Komplexität oder eine intrinsisch ungeordnete Proteinregion entweder in voller Länge oder trunkiert oder andere gefaltete Proteine aufweist, die bekanntermaßen Selbstinteraktionen fördern.

7. Verfahren nach Anspruch 5, ferner umfassend Erfassen oder Messen der gerichteten Kondensation, um den mechanischen Zustand des Genoms zu detektieren.

8. Verfahren nach Anspruch 7, wobei das Detektieren des mechanischen Zustands des Genoms Detektieren des Vorhandenseins von eng kondensiertem Heterochromatin beinhaltet.

9. Verfahren nach einem der Ansprüche 5-8, wobei Induzieren einer gerichteten Kondensation an den spezifischen genomischen Target-Loci mindestens eine Veränderung bewirkt, die aus der Gruppe ausgewählt ist, die aus Induzieren mechanischer Spannungen im Genom, Entspannen mechanischer Spannungen im Genom und Verändern mechanischer Spannungen im Genom besteht.

10. Verfahren nach einem der Ansprüche 5-9, wobei das Induzieren einer gerichteten Kondensation Induzieren von Enhancer-Promoter-Interaktionen, Induzieren von Super-Enhancer-Clustern, Aktivieren verwandter Arten von abstandsabhängigen genregulatorischen Elementen, Induzieren von Heterochromatisierung oder eine Kombination davon beinhaltet.

11. Verfahren nach einem der Ansprüche 5-10, wobei das System ein erstes Proteinsystem, das einem ersten genomischen Locus zugeordnet ist, und ein zweites Proteinsystem beinhaltet, das einem zweiten genomischen Locus zugeordnet ist, und wobei Aussetzen des ersten und des zweiten Proteinsystems der mindestens einen Lichtwellenlänge bewirkt, dass sich Tröpfchen bilden und dann miteinander verschmelzen.

**12.** Verfahren nach Anspruch 11, wobei bei der Bildung der Tröpfchen der Abstand von Mitte zu Mitte der beiden Tröpfchen weniger als 3 Mikrometer beträgt.

**13.** Verfahren nach einem der Ansprüche 5-12, wobei die erste Struktur ein erstes Konstrukt beinhaltet, das an SunTag fusioniertes dCas9 umfasst, das an ein zweites Konstrukt gebunden ist, das einen Antikörper mit einkettigem variablem Fragment umfasst, der an eine Superfaltungsvariante von GFP (sfGFP) und iLID fusioniert ist, wobei der Antikörper mit einkettigem variablem Fragment ein Kognat für SunTag ist.

**14.** Verfahren nach einem der Ansprüche 5 bis 13, umfassend ferner Zulassen, dass die erste Struktur als multimeres Proteingerüst zum Binden der zweiten Struktur dient, und Auslösen der Flüssig--Flüssig-Phasentrennung durch Aussetzen der ersten Struktur mindestens einer vorbestimmten Lichtwellenlänge und Veranlassen der Selbstanordnung der ersten und zweiten Struktur zu einem multimeren Proteinkomplex.

**Revendications**

**1.** Système pour le regroupement contrôlé de protéines régulatrices de gènes à des locus génomiques cibles spécifiques, comprenant :

une première structure comprenant :

(i) un premier segment ayant une protéine de ciblage génomique à base de Cas fusionnée ou attachée à une ou plusieurs premières séquences, chaque première séquence comprenant au moins une séquence choisie dans le groupe constitué d'un récepteur photosensible, d'un récepteur chimiquement sensible et d'une protéine d'oligomérisation photosensible ; ou
(ii) un premier segment ayant une protéine de ciblage génomique à base de Cas fusionnée ou attachée à un ou plusieurs gènes codant pour au moins une protéine sensible à au moins une longueur d'onde de lumière et un second segment fusionné au premier segment, le second segment ayant au moins une séquence sélectionnée dans le groupe constitué d'une séquence de faible complexité (LCS), d'une région protéique intrinsèquement désordonnée (IDR), d'un domaine de liaison à l'acide nucléique synthétique ou naturel, et au moins une séquence répétable, la séquence répétable comprenant un lieur fusionné à au moins un gène supplémentaire codant pour au moins une protéine sensible à au moins une longueur d'onde de lumière ; et

une seconde structure comprenant une seconde séquence choisie dans le groupe constitué d'un partenaire apparenté de la protéine réceptrice photosensible, d'un partenaire apparenté de la protéine sensible aux produits chimiques et d'un domaine de dimérisation complémentaire d'un module de dimérisation, la seconde séquence étant fusionnée à au moins une protéine régulatrice de la transcription ayant une région protéique de pleine longueur ou tronquée de faible complexité ou intrinsèquement désordonnée,
le système protéique étant conçu pour une condensation ciblée au niveau des locus génomiques cibles spécifiques en exposant le système protéique à au moins une longueur d'onde de lumière prédéterminée, un produit chimique auquel le récepteur sensible aux produits chimiques est sensible, ou une combinaison de ceux-ci .

**2.** Système selon la revendication 1, dans lequel la première structure comprend une première construction comprenant une dCas9 fusionnée à SunTag attachée à une seconde construction comprenant un anticorps à fragment variable à chaîne unique fusionné à un variant superpliant de la GFP (sfGFP) et de l'iLID, l'anticorps à fragment variable à chaîne unique étant apparenté à Sun Tag.

**3.** Système selon l'une quelconque des revendications 1 ou 2, dans lequel la première structure comprend au moins une protéine rapporteuse, la deuxième séquence est fusionnée à la BRD4, la FUS ou la TAF15 de pleine longueur ou tronquée, ou toute combinaison de celles-ci.

**4.** Système selon l'une quelconque des revendications 1 à 3, dans lequel la ou les premières séquences comprennent une première séquence répétitive.

**5.** Procédé in vitro pour le regroupement contrôlé de protéines régulatrices de gènes au niveau des locus génomiques cibles spécifiques, comprenant les étapes consistant à :

(a) fournir au moins un locus génomique cible spécifique et un ARN à guide unique complémentaire ;

(b) fournir un système protéique dans une cellule vivante, le système comprenant soit :

(i) un premier segment ayant une protéine de ciblage génomique à base de Cas fusionnée ou attachée à une ou plusieurs séquences, chaque séquence comprenant un récepteur photosensible, un récepteur chimiquement sensible, une protéine d'oligomérisation ou une combinaison de ceux-ci ; ou

(ii) un premier segment ayant une protéine de ciblage génomique à base de Cas fusionnée ou attachée à au moins un gène codant pour au moins une protéine sensible à au moins une longueur d'onde de lumière et un second segment fusionné au premier segment, le second segment ayant au moins une séquence sélectionnée dans le groupe constitué d'une séquence de faible complexité (LCS), d'une région protéique intrinsèquement désordonnée (IDR), d'un domaine de liaison à l'acide nucléique synthétique ou naturel et d'au moins une séquence répétable, la séquence répétable comprenant un lieur fusionné à au moins un gène supplémentaire codant pour au moins une protéine sensible à au moins une longueur d'onde de lumière ; et

(c) comprenant la condensation ciblée au niveau des locus génomiques cibles spécifiques en exposant le système protéique à au moins une longueur d'onde de lumière prédéterminée, un produit chimique auquel le récepteur sensible aux produits chimiques est sensible, ou une combinaison de ceux-ci.

6. Procédé selon la revendication 5, dans lequel le système comprend en outre un second segment ayant un partenaire apparenté de la protéine réceptrice photosensible ou sensible aux produits chimiques fusionnée à au moins une protéine régulatrice de gène ayant une protéine de pleine longueur ou tronquée de faible complexité ou une région protéique intrinsèquement désordonnée, ou d'autres protéines repliées connues pour favoriser les auto-interactions.

7. Procédé selon la revendication 5, comprenant en outre la détection ou la mesure de la condensation ciblée pour détecter l'état mécanique du génome.

8. Procédé selon la revendication 7, dans lequel la détection de l'état mécanique du génome comprend la détection de la présence d'hétérochromatine étroitement condensée.

9. Procédé selon l'une quelconque des revendications 5 à 8, dans lequel l'induction d'une condensation ciblée au niveau des locus génomiques cibles spécifiques induit au moins un changement choisi dans le groupe constitué de l'induction de contraintes mécaniques dans le génome, de la relaxation des contraintes mécaniques dans le génome et de la modification des contraintes mécaniques dans le génome.

10. Procédé selon l'une quelconque des revendications 5 à 9, dans lequel l'induction d'une condensation ciblée comprend l'induction d'interactions amplificateur-promoteur, l'induction de grappes de super amplificateurs, l'activation de types apparentés d'éléments régulateurs de gènes dépendant de la proximité, l'induction d'une hétérochromatisation ou une combinaison de celles-ci.

11. Procédé selon l'une quelconque des revendications 5 à 10, dans lequel le système comprend un premier système protéique associé à un premier locus génomique, et un second système protéique associé à un second locus génomique, et dans lequel l'exposition des premier et second systèmes protéiques à au moins une longueur d'onde de lumière provoque la formation de gouttelettes, qui ensuite se fusionnent.

12. Procédé selon la revendication 11, dans lequel lorsque les gouttelettes sont formées, la séparation centre à centre des deux gouttelettes est inférieure à 3 microns.

13. Procédé selon l'une quelconque des revendications 5 à 12, dans lequel la première structure comprend une première construction comprenant une dCas9 fusionnée à SunTag attachée à une seconde construction comprenant un anticorps à fragment variable à chaîne unique fusionné à un variant superpliant de la GFP (sfGFP) et de l'iLID, l'anticorps à fragment variable à chaîne unique étant apparenté à Sun Tag.

14. Procédé selon l'une quelconque des revendications 5 à 13, comprenant en outre le fait de permettre à la première structure de servir d'échafaudage protéique multimère pour la liaison de la seconde structure ; et de déclencher une séparation de phases liquide-liquide en exposant la première structure à au moins une longueur d'onde de lumière prédéterminée et en provoquant l'autoassemblage des première et seconde structures en un complexe protéique multimérique.

*FIG. 1*

*FIG. 2A*

*FIG. 2B*

*FIG. 3*

*410*

*412*

| Light Sensitive Region | Light Sensitive Region |

| dCas9 | Scaffold | *414* |

| Light Sensitive Region | Light Sensitive Region | *416* |

*422*  *420*  *424*

| Cognate | IDR/LCS |

*FIG. 4A*

**430**

**433**

**432**

**435**

| dCas9 | Light Sensitive Region | IDR / LCS / Binding Domain |

**431** **434**

*FIG. 4B*

**440**

**443** **446** **448**

**442** **445** **447**

| dCas9 | Light Sensitive Region | Linker | Light Sensitive Region | Linker | Light Sensitive Region |

**441** **444**

*FIG. 4C*

500

120

110

105

FIG. 5

**FIG. 6**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62621182
- US 62734063 B

- WO 2016011070 A

**Non-patent literature cited in the description**

- **YONGDAE SHIN et al.** Cell. Epub, 29 December 2016, vol. 168, 159-171